# EUROPÄISCHE PATENTSCHRIFT

(11) **EP 1 720 473 B1**
(45) Veröffentlichungstag und Bekanntmachung des Hinweises auf die Patenterteilung: **28.08.2013**
(21) Anmeldenummer: 04716528.7
(22) Anmeldetag: 03.03.2004
(51) Int. Cl.: A61B 17/74, A61B 17/86

(54) **KNOCHENFIXIERUNGSMITTEL**
BONE FIXATION MEANS
SYSTEME DE FIXATION D'OS

(43) Veröffentlichungstag der Anmeldung: 15.11.2006
(73) Patentinhaber: Synthes GmbH, 4436 Oberdorf (CH)
(72) Erfinder: DUTOIT, Christof, CH-4500 Solothurn (CH); FÜRST, Christoph, CH-4528 Zuchwil (CH)
(74) Vertreter: Lusuardi, Werther
(86) Internationale Anmeldenummer: PCT/CH2004/000119
(87) Internationale Veröffentlichungsnummer: WO 2005/084568

(56) Entgegenhaltungen:
- EP-A- 0 655 224
- WO-A-02/00123
- WO-A-98/05263
- US-A- 5 522 817
- US-A- 5 772 662
- US-A- 5 827 285
- US-A- 6 022 352

## Beschreibung

Die Erfindung bezieht sich auf ein Knochenfixationsmittel gemäss dem Oberbegriff des Patentanspruchs 1.

Solche Knochenfixationsmittel werden beispielsweise zur Versorgung von Frakturen am proximalen Femur, insbesondere von trochantären und Schenkelhalsfrakturen, eingesetzt. Solche Vorrichtungen umfassen im wesentlichen eine am Femur fixierbare Knochenplatte mit einer abgewinkelten, in den Schenkelhals einzubringenden Hülse zur rotationsstabilen Aufnahme der Schaftes des Knochenfixationsmittels.

Die bekannten Hüftschrauben zur Verwendung mit am Femur befestigbaren Knochenplatten haben alle den Nachteil, dass ihr in die Hülse der Knochenplatte einzuführender unrunder Schaft fest mit dem Verankerungselement (Schraube oder Klinge) des Knochenfixationsmittels verbunden ist.

Wenn nach erfolgter Implantation der Hüftschraube, der Chirurg die unrunde Hülse der Knochenplatte über den unrunden Schaft der Hüftschraube führen will, dann steht die Knochenplatte der DHS (dynamische Hüftschraube) nicht parallel zum Femur sondern muss dann in diese Lage gedreht werden, wobei die in der Hülse gefangene Hüfschraube mitrotiert. Je nachdem wird somit die Hüftschraube weiter nach vorn eingeschraubt oder wieder ein Stück zurückgeschraubt. Insbesondere bei Hüftschrauben, welche ein steiles Gewinde aufweisen, sogenannte Helix-Schrauben, ist dieser Vortrieb, bzw. Rückzug unzulässig gross.

Bei Hüftschrauben mit konventionellem, flachem Gewinde kann der Schaft der Schraube problemlos so ausgerichtet werden, dass die Hülsenlasche, wenn sie über den unrunden Schaft der Schraube geschoben wird, automatisch parallel zum Femur liegt. Die Platte wird normalerweise nicht mehr gedreht, nachdem sie über die Hülse gestülpt wurde.

Bei Hüftschrauben mit einem steilen Gewinde (Helix-Schraube) ist diese Ausrichtung schwierig, da bei kleiner Rotation der Schraube diese eine grosse Distanz weiter voroder zurückgeschraubt wird.

Aus der US 5,772,662 CHAPMAN ET AL. ist eine Knochenfixationsvorrichtung bekannt, welche einen Schaft und ein daran rotativ und axial fixierbares Verankerungselement umfasst. Die rotative Blockierung des Verankerungselements am Schaft ist bei dieser bekannten Vorrichtung jedoch nur durch axiale Verschiebung des Verankerungselement relativ zum Schaft lösbar oder blockierbar, wobei gleichzeitig auch die axiale Arretierung zwischen Schaft und Verankerungselement gelöst oder blockiert wird.

Hier will die Erfindung Abhilfe schaffen. Der Erfindung liegt die Aufgabe zugrunde, ein Knochenfixationsmittel zu schaffen, welches zwischen Schaft und Verankerungselement des Knochenfixationsmittels eine lösbar blockierbare Kupplung umfasst, so dass wahlweise der Schaft relativ zum Verankerungselement um die Längsachse rotierbar oder rotativ blockierbar Ist.

Die Erfindung löst die gestellte Aufgabe mit einem Knochenfixationsmittel, welches die Merkmale des Anspruchs 1 aufweist.

Die durch die Erfindung erreichten Vorteile sind im wesentlichen die folgenden:
- Die vorliegende Erfindung erlaubt die Verwendung von Knochenfixationsmitteln mit neuartigen Verankerungselementen, wie beispielsweise Hüftschrauben mit einem steilen Gewinde (Helix), in Kombination mit einer herkömmlichen Hülsenlasche, wie sie bereits seit über zwanzig Jahre auf dem Markt sind.
- Die Kompatibilität zwischen dem neuen Knochenfixationsmitteln und den herkömmlichen Hülsenlaschen erlaubt dem Chirurgen, sich intraoperativ zwischen herkömmlichen Knochenschrauben und dem neuen Knochenfixationsmittel zu entscheiden.

Erfindungsgemäß umfassen die Knochenfixationsmittel axiale Arretiermittel, mittels welcher der Schaft und das Verankerungselement axial zusammengehalten werden. Damit ist der Vorteil erreichbar, dass während der Implantation des Knochenfixationsmittels das Verankerungselement relativ zum Schaft rotierbar sein kann und dennoch die beiden Teile axial zusammengehalten werden und nicht auseinander fallen können.

Diese axialen Arretiermittel und die zusammenwirkenden Mittel können teilweise oder ganz aus denselben Elementen realisiert werden oder aber auch unabhängig voneinander ausgestaltet sein. Ferner können die axialen Arretiermittel lösbar oder fest ausgebildet sein. Die axialen Arretiermittel können beispielsweise einschnappbar sein und radial elastische Zungen mit in Nuten einschnappbaren Erhebungen umfassen, so dass ein einfaches Zusammenfügen zwischen Schaft und Verankerungselement erreichbar ist.

In einer anderen Ausführungsform sind die Zungen am Verankerungselement und die Nute am Schaft angeordnet. Die Erhebungen sind vorzugsweise konvex und in eine ringförmige Nute einrastbar.

In wiederum einer anderen Ausführungsform umfassen die axialen Arretiermittel zwei diametral die Hohlraumwand am hinteren Ende des Verankerungselementes durchdringende Stifte, deren Spitzen in eine zur Längsachse konzentrische Nute am Schaft eingreifen. Damit ist eine fertigungstechnisch einfache Ausführung der axialen Arretiermittel erreichbar.

In einer weiteren Ausführungsform sind an der Hohlraumwand am hinteren Ende des Verankerungselementes und am Schaft zur Längsachse konzentrische, fluchtende Ringnuten zur Aufnahme eines Sprengringes angebracht. Auch hier sind die axialen Arretiermittel einfach herstellbar.

Die zusammenwirkenden Mittel können reibschlüssig oder formschlüssig ausgestaltet sein. Im Falle einer reibschlüssigen Ausgestaltung eignen sich beispielsweise:
- Konuselemente, welche in einer komplementär konischen Bohrung verkeilbar sind. In einer Ausführungsform werden die Konuselemente eingeschlagen. In einer weiteren Ausführungsform werden die Konuselemente mit Hilfe eines Schraubmechanismus verkeilt. In wiederum einer anderen Ausführungsform sind das Konuselement mit einem konischen Aussengewinde und die konische Bohrung mit einem Innengewinde versehen. Einschlagbare Konuselemente ermöglichen eine einfache Ausgestaltung der Kupplung während die zuletzt beschriebene Konusverbindung eine einfach lösbare Variante darstellt; oder
- radial elastische Zungen, welche beispielsweise am Verankerungselement angebracht sind und mittels einer konischen Schraube gegen die Wand der Zentralbohrung im Schaft pressbar sind. Vorteilhaft ist hier die einfache Bedienbarkeit der zusammenwirkenden Mittel.

Im Falle einer formschlüssigen Ausgestaltung der zusammenwirkenden Mittel eignen sich beispielsweise am Schaft und am Verankerungselement, vorzugsweise stirnseitig angebrachte und ineinander in Eingriff bringbare Verzahnungen.

In einer Ausführungsform ist eine der Verzahnungen fest am Verankerungselement angebracht, während die zweite Verzahnung an einem am Schaft rotativ arretierten, axial verschiebbaren Fixationselement angebracht ist. Das Fixationselement kann mittels einer vom freien Ende des Schaftes her drehbaren Schraube axial verschoben werden.

In einer anderen Ausführungsform ist eine der Verzahnungen an einem rotativ arretierten Ringelement am Schaft angeordnet, während die zweite Verzahnung an einem ebenfalls rotativ arretierten, aber axial verschiebbaren Fixationselement angebracht ist. Das Fixationselement kann analog mittels einer vom freien Ende des Schaftes her drehbaren Schraube axial verschoben werden bis die Verzahnungen im Eingriff stehen oder voneinander entfernt sind.

Das Verankerungselement kann als Schraube mit einer Gewindesteigung G grösser als 50 mm, vorzugsweise grösser als 80 mm ausgebildet sein.

Die Anwendung einer Helix-Schraube hat gegenüber einer konventionellen Hüftschraube zahlreiche klinische Vorteile:
a) Die grössere Auflagefläche vermeidet ein Ausschneiden der Schraube aus dem Knochen.
b) Wird die Helix-Schraube in den Hüftkopf eingeschlagen, so wird das Knochenmaterial um das Implantat komprimiert, was das Risiko, dass das die Schraube aus dem Knochen ausschneidet, zusätzlich minimiert.
c) Im Gegensatz zur herkömmlichen Schraube verhindert die Helix-Schraube ein Rotieren des Hüftkopfes auf dem Implantat.

In einer weiteren Ausführungsform ist der Schaft des Knochenfixationsmittels in einem zur Längsachse orthogonalen Querschnitt betrachtet unrund ausgebildet. Damit ist der Vorteil erreichbar, dass die Hülsenlasche über den unrunden Schaft des Knochenfixationsmittels gestülpt werden kann, unabhängig davon wie weit das Knochenfixationsmittel in den Hüftkopf eingebracht wurde. Das Knochenfixationsmittel kann so in optimaler Position im Hüftkopf eingebracht und dann rotationsstabil verankert werden.

Die Fixationsvorrichtung für die Osteosynthese umfasst in einer Ausführungsform eine am Femur befestigbare Knochenplatte mit einer winklig anschliessenden Hülse, welche zur Aufnahme des Schaftes des Knochenfixationsmittels geeignet ist. Vorzugsweise sind der Schaft aussen und die Hülse innen mit komplementär unrunden Querschnitten ausgestaltet.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1a eine Seitenansicht auf eine Ausführungsform der erfindungsgemässen Knochenfixationsmittel;
Fig. 1b eine Seitenansicht auf eine weitere Ausführungsform der erfindungsgemässen Knochenfixationsmittel;
Fig. 1c eine Seitenansicht auf wiederum eine weitere Ausführungsform der erfindungsgemässen Knochenfixationsmittel;
Fig. 1d eine Seitenansicht auf eine andere Ausführungsform der erfindungsgemässen Knochenfixationsmittel;
Fig. 2a eine schematische Darstellung der axialen Arretiermittel der in Fig. 1a dargestellten Ausführungsform der erfindungsgemässen Knochenfixationsmittel;
Fig. 2b eine schematische Darstellung der axialen Arretiermittel der in Fig. 1 b dargestellten Ausführungsform der erfindungsgemässen Knochenfixationsmittel;
Fig. 2c eine schematische Darstellung der axialen Arretiermittel der in Fig. 1 c dargestellten Ausführungsform der erfindungsgemässen Knochenfixationsmittel;
Fig. 2d eine perspektivische Darstellung einer Ausführungsform eines Sprengringes der in Fig. 2c dargestellten axialen Arretiermittel;
Fig. 2e einen Schnitt durch eine andere Ausführungsform eines Sprengringes für die in Fig. 2c dargestellte Ausführungsform der erfindungsgemässen Knochenfixationsmittel;
Fig. 2f eine Ansicht auf die in Fig. 2e dargestellte Ausführungsform eines Sprengringes;
Fig. 2g einen Schnitt durch eine weitere Ausführungsform eines Sprengringes für die in Fig. 2c dargestellte Ausführungsform der erfindungsgemässen Knochenfixationsmittel;
Fig. 2h einen Ansicht auf die in Fig. 2g dargestellte Ausführungsform eines Sprengringes;
Fig. 2i eine schematische Darstellung der axialen Arretiermittel der in Fig. 1d dargestellten Ausführungsform der erfindungsgemässen Knochenfixationsmittel;
Fig. 2k einen zur Längsachse orthogonalen Schnitt durch die Hülse der in Fig. 2i dargestellten axialen Arretiermittel;
Fig. 3a einen vergrösserten Ausschnitt des Kreises A in Fig. 1 a;
Fig. 3b einen zur Fig. 3a analogen Ausschnitt aus einer anderen Ausführungsform der erfindungsgemässen Knochenfixationsmittel;
Fig. 3c einen Längsschnitt durch das Knochenverankerungselement und das feste Ende des Schaftes einer anderen Ausführungsform der erfindungsgemässen Knochenfixationsmittel;
Fig. 3d einen Längsschnitt durch das Knochenverankerungselement und das feste Ende des Schaftes einer weiteren Ausführungsform der erfindungsgemässen Knochenfixationsmittel;
Fig. 3e eine perspektivische Ansicht des Knochenverankerungselementes wiederum einer weiteren Ausführungsform der erfindungsgemässen Knochenfixationsmittel;
Fig. 3f einen zur Fig. 3a anlogen Ausschnitt aus wiederum einer anderen Ausführungsform der erfindungsgemässen Knochenfixationsmittel;
Fig. 3g eine perspektivische Darstellung des Keilelementes der in Fig. 3d dargestellten Ausführungsform der erfindungsgemässe Knochenfixationsmittel;
Fig. 4a einen zur Fig. 3a analogen Ausschnitt aus einer weiteren Ausführungsform der erfindungsgemässen Knochenfixationsmittel;
Fig. 4b einen Längsschnitt durch das Knochenverankerungselement und das feste Ende des Schaftes einer weiteren Ausführungsform der erfindungsgemässen Knochenfixationsmittel;
Fig. 4c einen Längsschnitt durch das Knochenverankerungselement und das feste Ende des Schaftes einer anderen Ausführungsform der erfindungsgemässen Knochenfixationsmittel;
Fig. 4d einen Längsschnitt durch eine weitere Ausführungsform der erfindungsgemässen Knochenfixationsmittel; und
Fig. 5 einen teilweisen Schnitt durch die im Femur implantierte erfindungsgemässe Fixationsvorrichtung.

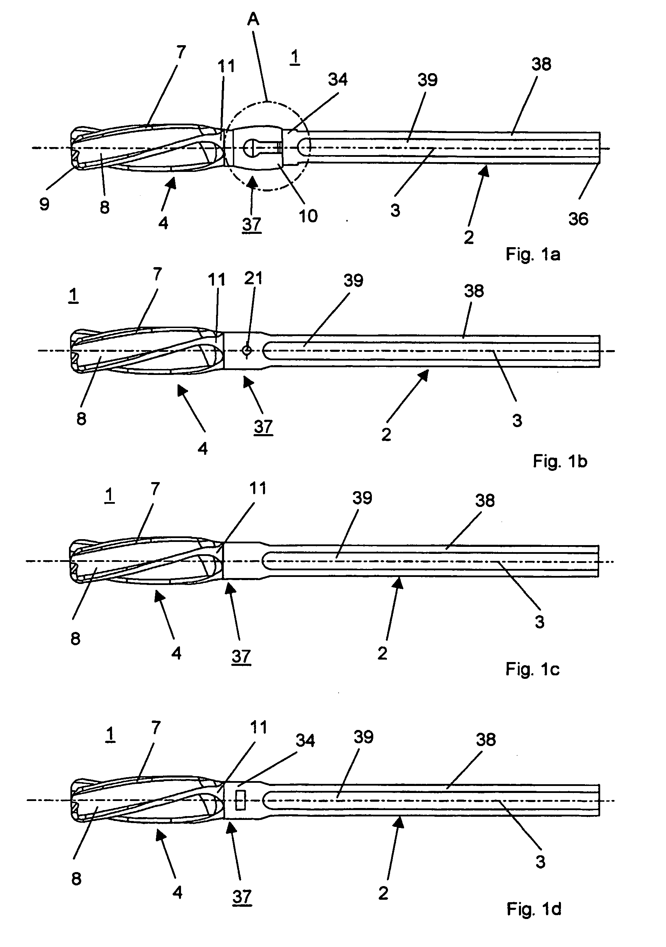

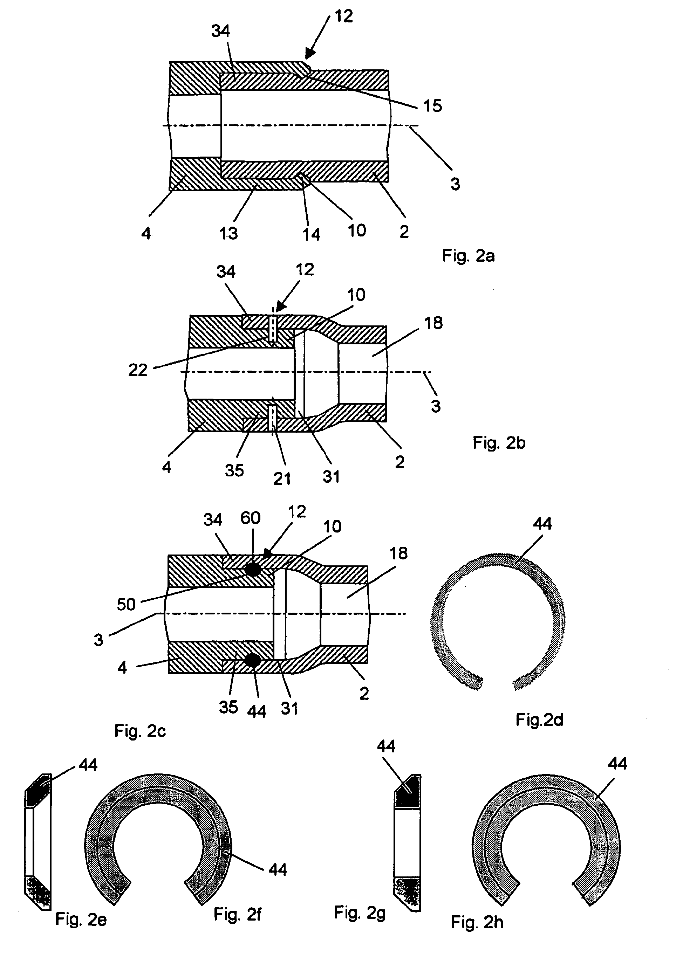

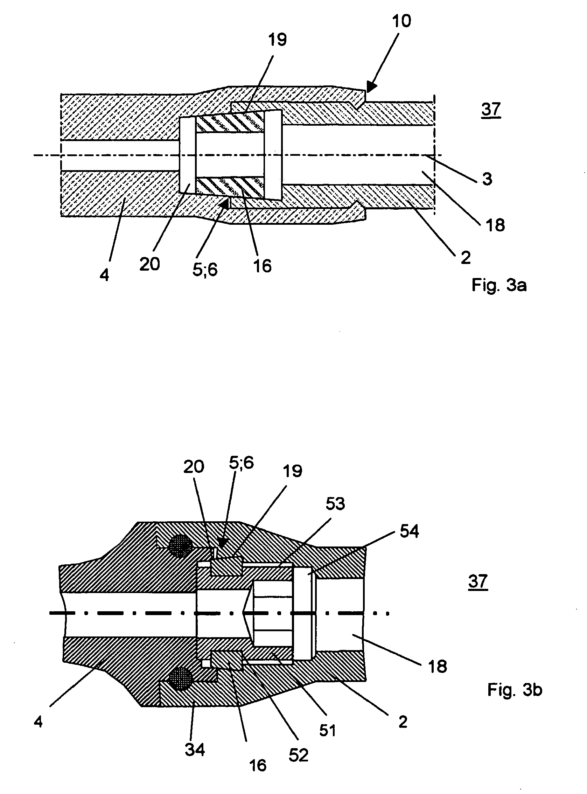

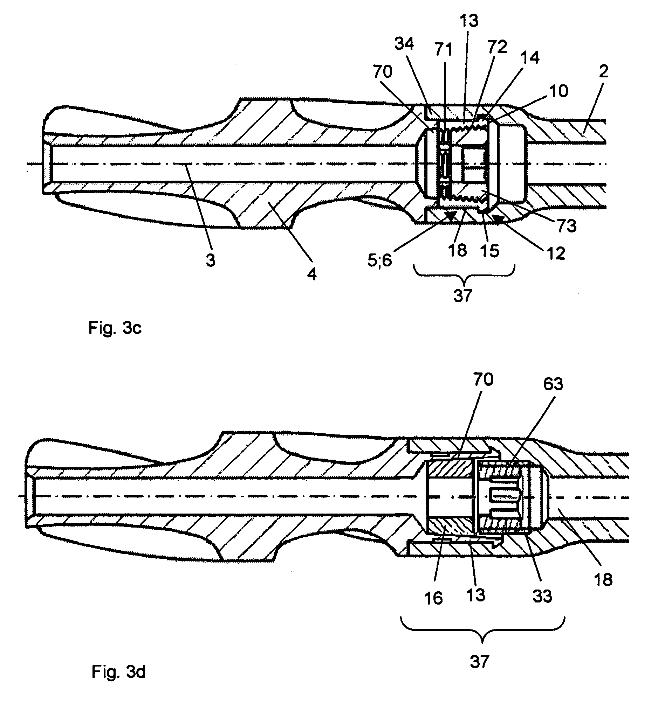

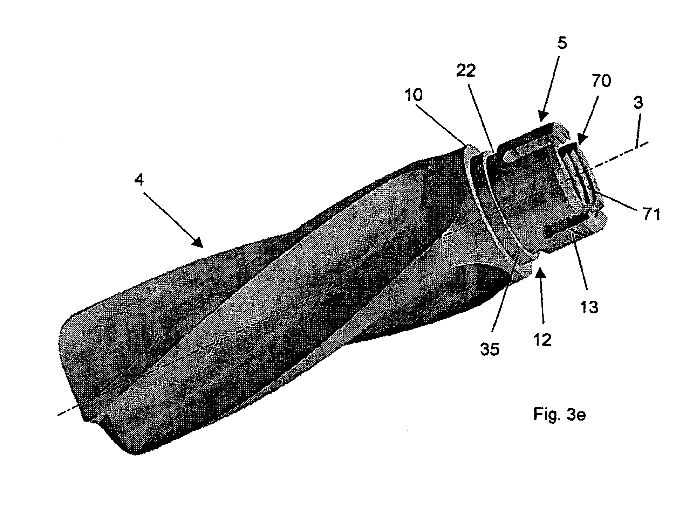

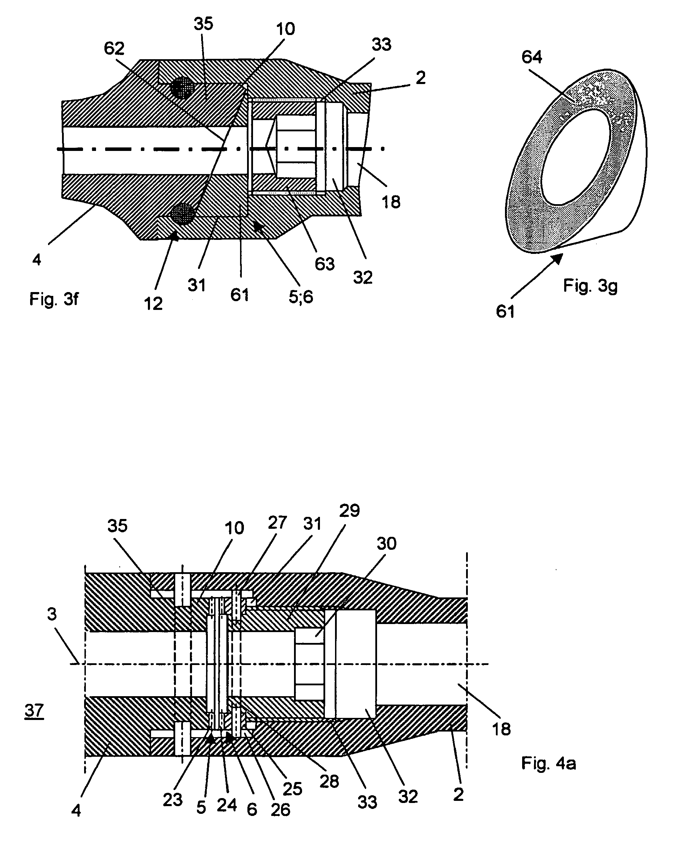

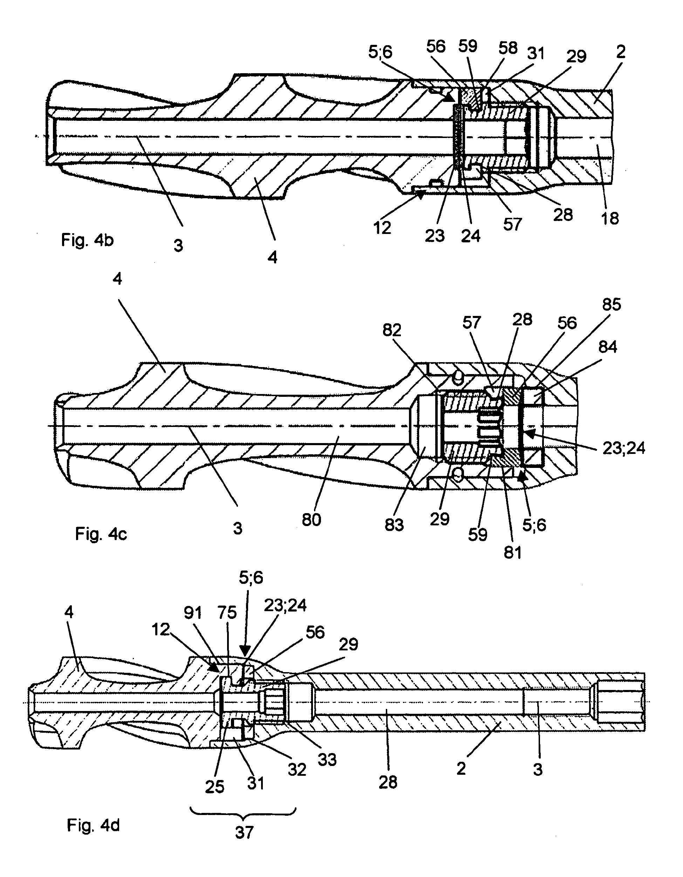

In den Figuren 1 bis 4 sind verschiedene Ausführungsformen der erfindungsgemässen Knochenfixationsmittel 1 mit einer Kupplung 37 zwischen Schaft 2 und Verankerungselement 4 dargestellt, wobei die Kupplung 37 axiale Arretiermittel 12 (Fig. 2a bis 2k) und eine rotative Arretierung ermöglichende, zusammenwirkende Mittel 5;6 (Fig. 3;4) umfasst. Dabei können die zusammenwirkenden Mittel 5;6 eine reibschlüssige Blockierung (Fig. 3a bis 3g) oder eine formschlüssige Blockierung (Fig. 4a bis 4d) der relativen Rotationsbewegung zwischen Schaft 2 und Verankerungselement 4 ermöglichen.

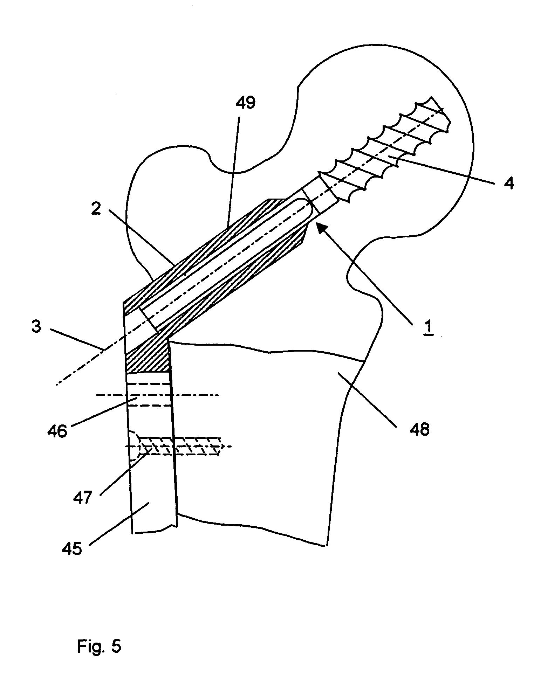

Das Verankerungselement 4 ist in den in Figur 1a bis 1d dargestellten Ausführungsformen als zur Längsachse 3 koaxiale Spiralklinge mir vier auf dem Umfang verteilten, schraubenlinienförmigen Klingen 7 ausgestaltet, wobei die Vertiefung 8 zwischen den Klingen 7 am freien Ende 9 des Verankerungselementes 4 offen sind. Die radiale Tiefe der Vertiefungen 8 bleibt auf einer axialen Länge L konstant und nimmt dann kontinuierlich ab, bis die Vertiefungen 8 am festen Ende 10 des Verankerungselementes 4 in dessen Mantelfläche 11 übergehen. Der Schaft 2 ist auf seiner Mantelfläche 38 mit zwei diametral angeordneten, zur Längsachse 3 parallelen Abflachungen 39 versehen, so dass der Schaft 2 in einer komplementären Bohrung einer Hülse 49 (Fig. 5) gegen Rotation um die Längsachse 3 gesichert werden kann.

In den Fig. 2a bis 2k sind detailliert Ausführungsformen der zur Kupplung 37 (Fig. 1) zählenden, axialen Arretiermittel 12 dargestellt. Die axialen Arretiermittel 12 bewirken nur eine axiale Fixierung zwischen Schaft 2 und Verankerungselement 4, während die Rotation des Verankerungselementes 4 relativ zum Schaft 2 nicht behindert wird.

Bei der in Figur 2a abgebildeten Ausführungsform der axialen Arretiermittel 12 umfassen diese vier am festen Ende 10 des Verankerungselementes 4 angeordnete, auf dem Umfang gleichmässig verteilte und zur Längsachse 3 parallele Zungen 13, welche axial über das feste Ende 34 des Schaftes 2 schiebbar und mit dem Schaft 2 verbindbar sind. Diese Zungen 13 sind quer zur Längsachse 3 elastisch deformierbar und weisen innen radiale Erhebungen 14 auf, welche in eine sich über dem gesamten Umfang des Schaftes 2 erstreckende Nute 15 mit V-förmigem Querschnitt einrastbar sind. Die axialen Arretiermittel 12 bewirken eine axiale Fixierung des Schaftes 2 relativ zum Verankerungselement 4 in beiden Richtungen, behindern jedoch die freie Rotation des Verankerungselementes 4 relativ zum Schaft 2 um die Längsachse 3 nicht.

Die in Figur 2b abgebildete Ausführungsform der axialen Arretiermittel 12 umfasst zwei Arretierstifte 21, welche am festen Ende 10 des Schaftes 2 radial in die Wand des Schaftes 2 eingebracht sind, und deren Spitzen radial in das erste erweiterte Segment 31 der Zentralbohrung 18 im Schaft 2 ragen und dort in einem aussen peripher auf dem Zapfen 35 umlaufenden Einstich 22 axial gefangen sind. Da sich dieser Einstich 22 über den gesamten Umfang des Zapfens 35 erstreckt, wird die Rotation des Schaftes 2 relativ zum Verankerungselement 4 durch die zwei Arretierstifte 21 nicht behindert.

Bei der in Figur 2c abgebildeten Ausführungsform sind die axialen Arretiermittel 12 durch einen in zwei konzentrischen Ringnuten 50;60 eingefügten Sprengring 44 realisiert. Die Ringnuten 50;60 sind derart angeordnet, dass aussen am Zapfen 35 analog zur in Fig. 2b dargestellten Ausführungsform eine peripher umlaufende, erste Ringnut 50 und im erweiterten Segment 31 der Zentralbohrung 18 im Schaft 2 eine zweite, peripher umlaufende Ringnut 60 angeordnet sind. Der in diese zwei Ringnuten 50;60 eingelegte Sprengring 44 verhindert eine axiale Relativbewegung zwischen Verankerungselement 4 und Schaft 2 während eine relative Rotationsbewegung dieser zwei Teile um die Längsachse 2 als Drehachse weiterhin möglich ist. Der Sprengring 44 kann in verschiedenen Ausführungsformen vorliegen. Beispielsweise kann die orthogonal zur Längsachse 3 betrachtete Querschnittsfläche des Sprengringes 44 kreisrund (Fig. 2c), rechteckig (Fig. 2d), abgeschrägt (Fig. 2e bis 2h) oder auch abgestuft sein.

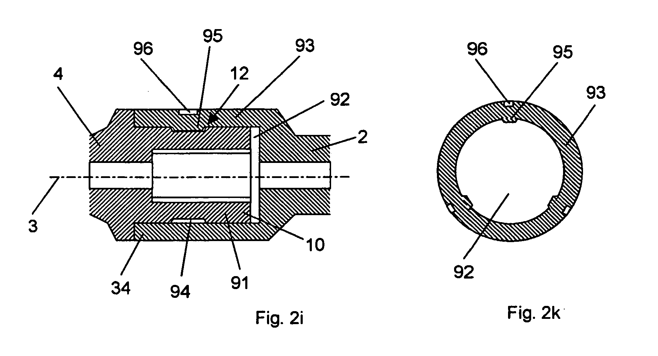

In der in den Fig. 2i und 2k dargestellten Ausführungsform sind die Arretiermittel 12 dadurch realisiert, dass das feste Ende 10 des Verankerungselementes 4 ein zur Längsachse 3 koaxiales, kreiszylindrisches Anschlussstück 91 umfasst, welches in einer ebenfalls zur Längsachse 3 koaxialen, kreiszylindrischen Öffnung 92 einer am festen Ende 34 des Schaftes 2 angeordneten Hülse 93 passend aufgenommen wird. Das Anschlussstück 91 ist mit einer sich über den gesamten Umfang erstreckenden Kerbe 94 versehen. Nach dem Einführen des Anschlussstückes 91 in die Öffnung 92 werden an der Wand der Hülse 93 durch plastische Verformung mehrere, vorzugsweise drei Dellen 95 angebracht. Die Dellen 95 verengen die zur Längsachse 3 orthogonale Querschnittsfläche der Öffnung 92 und greifen in die Kerbe 94 ein. Durch Anbringen von Einprägungen 96 an der Wand der Hülse 93 vor dem Zusammenfügen mit dem Schaft 2 kann die Form der Dellen 95 so gesteuert werden, dass das Anschlussstück 91 relativ zur Hülse 93 axial verriegelt ist, dass aber das Anschlussstück 91 in der Hülse 93 trotzdem um die Längsachse 3 rotierbar gelagert ist.

Die Kupplung 37 (Fig. 1) umfasst neben den axialen Arretiermitteln 12 (Fig 2a bis 2k) die zusammenwirkenden Mittel 5;6, mittels welcher die relative Rotation um die Längsachse 3 zwischen Verankerungselement 4 und Schaft 2 wahlweise blockierbar oder deblockierbar sind.

In Figur 3a bis 3g sind verschiedene Ausführungsformen von Kupplungen 37 abgebildet, welche eine reibschlüssige Blockierung der relativen Rotationsbewegung von Schaft 2 und Verankerungselement 4 ermöglichen.

Fig. 3a zeigt eine nicht erfindungsgemässe Ausführungsform. In Figur 3a umfasst die Kupplung 37 als erstes zusammenwirkendes Mittel 5 ein zur Längsachse 3 koaxiales Konuselement 16 und als zweites zusammenwirkendes Mittel 6 eine zum Konuselement 16 komplementär konische Bohrung 17 im Schaft 2 und im Verankerungselement 4. Dabei ist der Übergang zwischen dem ersten, Im Schaft 2 angeordneten Innenkonussegment 19 der Bohrung 18 und einem zweiten, im Verankerungselement 4 angeordneten Innenkonussegment 20 kontinuierlich. Das Konuselement 16 ist in den Konussogmenten 19;20 axial verschiebbar gelagert. Die Innenkonussegmente 19;20 sowie das Konuselement 16 erweitern sich gegen das feste Ende 10 des Verankerungselementes 4. Mittels eines durch die Zentralbohrung 18 im Schaft 2 durchführbaren Bolzen (nicht gezeichnet) kann das Konuselement 16 mit Hilfe eines zusätzlichen Instrumentes, zum Beispiel einem Hammer gegen das freie Ende 9 (Fig. 1) des Verankerungselementes 4 gepresst werden, bis es sich in den zwei Innenkonussegmenten 19;20 verkeilt. Um das Verkeilen unter möglichst geringem Kraftaufwand zu ermöglichen, ist das Konuselement 16 in Längsrichtung geschlitzt. Da sich das Konuselement 16 in beiden Innenkonussogmenten 19;20 verkeilt, sind in der verkeilten Position des Konuselementes 16 der Schaft 2 und das Verankerungselement 4 reibschlüssig miteinander verbunden und gegen eine relative Rotation blockiert. Das Konuselement 16 kann jedoch in dieser Ausführungsform nicht wieder gelöst werden.

Die in Figur 3b abgebildete Ausführungsform der zusammenwirkenden Mittel 5;6 unterscheiden sich von der in Fig. 3a dargestellten Ausführungsform der zusammenwirkenden Mittel 5;6 nur darin, dass das Konuselement 16 anstatt mit Hilfe eines zusätzlichen Instrumentes (Fig. 3a) mit Hilfe eines in Schaft 2 eingebrachten Schraubenelementes 51 gegen das freie Ende 9 (Fig. 1) des Verankerungselementes 4 gepresst werden kann. Das Konuselement 16 ist auf seiner gesamten Länge in einer peripher umlaufenden Nute 52 am Schraubenelement 51 axial gefangen, kann jedoch relativ zum Schraubenelement 51 frei rotieren. Das Schraubenelement 51 ist in ein Innengewinde 53 einer am festen Ende 34 des Schaftes 2 angeordneten, zylindrischen Erweiterung 54 der Bohrung 18 einschraubbar.

In Fig. 3c ist eine Ausführungsform der Kupplung 37 dargestellt, deren zusammenwirkende Mittel 5;6 reibschlüssig blockierbar sind. Die axialen Arretiermittel 12 sind analog zu der in Fig. 2a dargestellten Ausführungsform als radial federbare Zungen 13 ausgebildet, unterscheiden sich jedoch dadurch, dass sie nicht über das feste Ende 34 des Schaftes 2 geschoben werden, sondern in die Zentralbohrung 18 im Schaft 2. Die radialen Erhebungen 14 sind aussen an den Zungen 13 und die Nute 15 in der Zentralbohrung 18 angeordnet. Die Zungen 13 umschliessen einen zur Längsachse 3 koaxialen Hohlraum 70, dessen Wandung ein konisches Innengewinde 71 aufweist, in welches eine ein komplementäres Aussengewinde 72 aufweisende Klemmschraube 73 schraubbar ist. Beim Anziehen der Klemmschraube 73 werden die als erstes zusammenwirkendes Mittel 5 geeigneten Zungen 13 radial gegen die als zweites zusammenwirkendes Mittel 6 geeignete Wand der Bohrung 18 gepresst, wodurch der Schaft 2 rotativ reibschlüssig mit dem Verankerungselement 4 verbunden wird. Da die Erhebungen 14 an den Zungen 13 auch bei nicht gespreizten Zungen 13 in die Nute 15 eingreifen sind die Wirkungen der axialen Arretiermittel 12 und der zusammenwirkenden Mittel 5;6 unabhängig voneinander. Hingegen sind die axialen Arretiermittel 12 und die zusammenwirkenden Mittel 5;6 hier nicht unabhängig voneinander ausgebildet.

Die in Fig. 3d dargestellte Ausführungsform der Kupplung 37 unterscheidet sich von der in Fig. 3c dargestellten Ausführungsform nur darin, dass der von den Zungen 13 umschlossene, konische Hohlraum 70 eine glatte Wand aufweist, so dass ein komplementär konisches Konuselement 16 im Hohlraum 70 verkeilbar ist. Das Konuselement 16 wird mittels einer Spannschraube 63, welche in ein in der Bohrung 18 angeordnetes Innengewinde 33 schraubbar ist, axial in den Hohlraum 70 gepresst.

Bei der in Fig. 3e dargestellten Ausführungsform sind die axialen Arretiermittel 12 und zusammenwirkenden Mittel 5; 6 unabhängig voneinander ausgestaltet. Die axialen Arretiermittel 12 sind analog zu der in Fig. 2b dargestellten Ausführungsform ausgebildet, d.h. am festen Ende 10 des Verankerungselementes 4 ist ein Zapfen 35 mit einem zur Längsachse 3 konzentrischen Einstich 22 zur radialen Aufnahme von Arretierstiften 21 (Fig. 2b) angeordnet. Die Ausgestaltung der zusammenwirkenden Mittel 5; 6 unterscheidet sich von der in Fig. 3c dargestellten Ausführungsform nur darin, dass die ersten zusammenwirkenden Mittel 5 endständig am Zapfen 35 angeordnet sind und radial elastische Zungen 13 ohne Erhebungen 14 umfassen.

Die in den Fig. 3f und 3g dargestellte Ausführungsform unterscheidet sich bezüglich der zusammenwirkenden Mittel 5; 6 von der in Fig. 3b dargestellten Ausführungsform darin, dass als erstes zusammenwirkendes Mittel 5 anstelle des Konuselementes 16 (Fig. 3a) ein asymmetrisches keilförmiges Klemmelement 61 in der ersten Erweiterung 31 der Zentralbohrung 18 des Schaftes 2 angeordnet ist. Dieses eine abgeschrägte Stirnfläche 64 aufweisende keilförmige Klemmelement 61 wird zur Blockierung gegen eine komplementäre Anschrägung 62 am Zapfen 35 des Klemmelementes 4 gepresst. Als zweites zusammenwirkendes Mittel 6 ist in dem hier ein Innengewinde 33 aufweisenden zweiten Erweiterung 32 der Zentralbohrung 18 im Schaft 2 eine Spannschraube 63 angeordnet, mittels welcher das keilförmige Klemmelement 61 gegen die Anschrägung 62 am festen Ende 10 des Verankerungselementes 4 pressbar ist. Die axialen Arretiermittel 12 sind analog zu der in Fig. 2c dargestellten Ausführungsform realisiert.

In den Figuren 4a bis 4d sind verschiedene Ausführungsformen von Kupplungen 37 abgebildet, welche zusammenwirkende Mittel 5; 6 für eine formschlüssige Blockierung der relativen Rotationsbewegung von Schaft 2 und Verankerungselement 4 umfassen.

Bei der in Figur 4a abgebildeten Ausführungsform der zusammenwirkenden Mittel 5; 6 weist das Verankerungselement 4 an seinem festen Ende 10 einen im Durchmesser verjüngten Zapfen 35 auf, welcher in ein erstes erweitertes Segment 31 in der Zentralbohrung 18 des Schaftes 2 axial einführbar ist und an seiner zur Längsachse 3 orthogonalen Stirnfläche eine erste Verzahnung 23 aufweist. Der mit der ersten Verzahnung 23 versehene Zapfen 35 bildet hier das erste der zusammenwirkenden Mittel 5, während das zweite der zusammenwirkenden Mittel 6 durch das im ersten erweiterten Segment 31 gelagerte, axial verschiebbare Fixationselement 56 gebildet wird. Das hier ringförmig ausgebildete Fixationsmittel 56 weist an seiner dem Verankerungselement 4 zugewandten Stirnfläche eine zweite Verzahnung 24 auf. Durch axiale Verschiebung dieses Fixationselementes 56 sind die zwei Verzahnungen 23;24 ineinander in Eingriff bringbar oder ausrastbar. Die Verschiebung des Fixationselementes 56 erfolgt durch eine Schraube 29, welche in dem im zweiten erweiterten Segment 32 angebrachten Innengewinde 33 durch Drehen im Uhrzeigersinn oder im Gegenuhrzeigersinn axial in beide Richtungen verschiebbar ist. Die Mittel 30 dienen zur Aufnahme eines Schraubendrehers und können beispielsweise als Sechskant- oder als Torx-Antrieb ausgebildet sein. Die Abmessungen sind derart bemessen, dass ein Schraubendreher (nicht gezeichnet) vom freien Ende 36 (Fig. 1) des Schaftes 2 durch die Zentralbohrung 18 im Schaft 2 durchführbar ist und mit den Mitteln 30 in Eingriff bringbar ist.

Das Fixationselement 56 ist axial in Richtung der Längsachse 3 verschiebbar, während die Rotation um die Längsachse 3 verhindert wird. Bei der in Figur 4a abgebildeten Ausführungsform umfasst das Fixationselement 56 zwei diametral angeordnete, durch die Wand des Fixationselementes 56 durchgehende Stifte 27, deren Spitzen in einer zweiten Ringnut 28 an der Schraube 29 axial gefangen sind, so dass die Schraube 29 um die Längsachse 3 rotierbar ist, während eine Rotation des Fixationselementes 56 um die Längsachse 3 durch die in den zwei zur Längsachse 3 parallele Längsnuten 26 in den Innenwand des ersten erweiterten Segmentes 31 geführten hinteren Enden der Stifte 27 verhindert wird.

Die in Fig. 4b dargestellte Ausführungsform der zusammenwirkenden Mittel 5;6 unterscheiden sich von der in Fig. 4a dargestellten Ausführungsform der zusammenwirkenden Mittel 5;6 nur darin, dass ein unrundes, in einem zur Längsachse 3 orthogonalen Querschnitt betrachtet vorzugsweise ovales Fixationselement 56 vorgesehen ist, welches in einem komplementär ausgestalteten, ersten erweiterten Segment 31 der Zentralbohrung 18 im Schaft 2 rotativ fixiert ist. Das Fixationselement 56 weist einen zur Längsachse 3 orthogonalen Einschnitt 57 auf, so dass das U-förmig ausgebildete Fixationselement 56 vor der Montage der Schraube 29 in die Bohrung 18 im Schaft 2 quer zur Längsachse 3 über die Schraube 29 schiebbar ist. Der Einschnitt 57 weist an seinem hinteren Ende 58 eine Verengung 59 auf, welche quer zur Längsachse 3 in die zweite Ringnut 28 einschiebbar ist. Das Fixationselement 56 ist durch diese in die zweite Ringnut 28 eingefügte Verengung 59 axialfest mit der Schraube 29 verbunden, während die Schraube 29 relativ zum Fixationselement 56 um die Längsachse 3 rotierbar ist.

In Fig. 4c ist eine Ausführungsform der zusammenwirkenden Mittel 5;6 dargestellt, welche sich von der in Fig. 4b dargestellten Ausführungsform nur darin unterscheidet, dass das Fixationselement 56 in einem endständig angeordneten, ersten erweiterten Segment 81 der Zentralbohrung 80 im Verankerungselement 4 aufgenommen wird. Im zur Längsachse 3 orthogonalen Querschnitt betrachtet, weisen das Fixationselement 56 sowie das erste erweiterte Segment 81 eine ovale Querschnittsfläche auf, so dass das Fixationselement 56 gegen Rotation um die Längsachse 3 gesichert, aber axial verschiebbar im ersten erweiterten Segment 81 aufgenommen wird. Das Fixationselement 56 weist analog zu Fig. 4b einen zur Längsachse 3 orthogonalen Einschnitt 57 auf, so dass das U-förmig ausgebildete Fixationselement 56 quer zur Längsachse 3 über die Schraube 29 schiebbar ist. Der Einschnitt 57 weist hier ebenfalls eine Verengung 59 auf, welche quer zur Längsachse 3 in die Ringnute 28 schiebbar ist, so dass das Fixationselement 56 axialfest mit der Schraube 29 verbunden ist, wobei die Rotation der Schraube 29 nicht behindert wird. Die Schraube 29 ist hier in ein Innengewinde 82, welches in einem zweiten erweiterten Segment 83 der Zentralbohrung 80 im Verankerungselement 4 angeordnet ist, schraubbar. Ferner ist ein ovales Ringelement 84 in einer komplementären, ovalen Ausnehmung 85 in der Zentralbohrung 18 im Schaft 2 angeordnet und durch die ovale Ausgestaltung von Ringelement 84 und Ausnehmung 85 ebenfalls gegen Rotation um die Längsachse 3 gesichert. Die beiden ineinander in Eingriff bringbaren Verzahnungen 23;24 sind an den benachbarten Stirnflächen am Fixationselement 56 und am Ringelement 84 angebracht, so dass die Verzahnungen 23;24 durch axiales Verschieben des Fixationselementes 56 mittels der Schraube 29 ein- respektive ausrastbar sind.

Die In Fig. 4d dargestellte Ausführungsform der zusammenwirkenden Mittel 5;6 unterscheiden sich von der in Fig. 4a dargestellten Ausführungsform der zusammenwirkenden Mittel 5;6 nur darin, dass ein unrundes, in einem zur Längsachse 3 orthogonalen Querschnitt betrachtet vorzugsweise ovales Fixationselement 56 vorgesehen ist, welches in einem komplementär ausgestalteten, ersten erweiterten Segment 31 der Zentralbohrung 18 im Schaft 2 rotativ fixiert ist. Am festen Ende 10 des Verankerungselementes 4 ist ein zur Längsachse 3 koaxiales kreiszylindrisches Anschlussstück 91 angeordnet, welches In eine am festen Ende 34 des Schaftes 2 angeordnete, komplementär ausgebildete zweite Erweiterung 32 der Zentralbohrung 18 einführbar ist. Dieses Anschlussstück 91 weist stirnseitig eine erste Verzahnung 23 aus, welche mit einer gegenüberliegend stirnseitig am Fixationselement 56 angeordneten, zweiten Verzahnung 24 in Eingriff bringbar ist. Dazu umfasst die Kupplung 37 eine Schraube 29, welche in ein Innengewinde 33 in der Zentralbohrung 18 einschraubbar ist. An ihrem dem Verankerungselement 4 zugewandten Ende weist die Schraube 29 einen einen grösseren Durchmesser aufweisenden Schraubenkopf 25 auf, welcher quer zur Längsachse 3 in eine einseitig radial offene Führung 75 einschiebbar ist und dort axialfest aber rotativ frei bewegbar befestigbar ist. Beim Anziehen der Schraube 29 wird also einerseits der Schaft 2 axial gegen das Verankerungselement 4 gepresst, wodurch von der Schraube 29 die Funktion der axialen Arretiermittel 12 übernommen wird, und andererseits werden die zwei Verzahnungen 23;24 in Eingriff gebracht, so dass In der hier dargestellten Ausführungsform die axialen Arretiermittel 12 und die zusammenwirkenden Mittel 5;6 nicht unabhängig voneinander ausgebildet sind.

Bei den in den Fig. 4a bis 4d abgebildeten Ausführungsformen der zusammenwirkenden Mittel 5;6 sind somit bei ineinander im Eingriff stehenden Verzahnungen 23;24 der Schaft 2 und das Verankerungselement 4 rotativ formschlüssig gekuppelt, während bei ausgerasteten Verzahnungen 23;24 der Schaft 2 relativ zum Verankerungselement 4 um die Längsachse 3 rotierbar ist.

Wie in Figur 5 dargestellt kann die Hülsenlasche 45 mittels in die Bohrungen 46 einzuführender Knochenschrauben 47 lateral am Femur 48 befestigt werden, währenddem die Führungshülse 49 lateral der Schenkelhals- oder trochantären Fraktur zu liegen kommt. Somit kann das Kopffragment mit Hilfe der Knochenfixationsmittel 1 mit dem Rest des Femurs 48 rotationsstabil fixiert werden.

Die Operationstechnik zur Implantation des Knochenfixationsmittels besteht darin, dass
- mittels eines Instrumentes in einem Arbeitsvorgang in latero-medialer Richtung unterhalb des grossen Trochanters mehrere Bohrungen unterschiedlichen Durchmessers zum Einbringen des Knochenfixationsmittels 1 und der an der Hülsenlasche 45 angebrachten Führungshülse 49 in das Zentrum des Schenkelhalses eingebracht werden;
- anschliessend das Knochenfixationsmittel 1 in den Schenkelhals eingedreht oder eingehämmert wird, wobei mittels eines Zielgerätes die korrekte Einschlag- oder Einschraubtiefe bestimmt wird,
- danach die Führungshülse 49 der Hülsenlasche 45 über den Schaft des Knochenfixationsmittels geschoben und am Femurschaft ausgerichtet wird;
- die Hülsenlasche 45 mit Hilfe von als Knochenschrauben ausgebildeten Knochenfixationsmitteln 20 am Knochenschaft fixiert wird; und
- mittels eines Instrumentes die Rotationsbewegung von Schaft 2 und Verankerungselement 4 blockiert wird.

## Patentansprüche

1. Knochenfixationsmittel (1) umfassend
A) einen longitudinalen Schaft (2) mit der Längsachse (3);
B) ein am Schaft (2) axial befestigbares, in einem Knochen fixierbares Verankerungselement (4) mit der identischen Längsachse (3); und
C) zusammenwirkende Mittel (5;6) am Schaft (2) und am Verankerungselement (4) vorgesehen sind, welche wahlweise eine Rotation des Verankerungselementes (4) um die Längsachse (3) relativ zum Schaft (2) zulassen oder blockieren, wobei
D) am Schaft (2) und am Verankerungselement (4) axiale Arretiermittel (12) angeordnet sind, wodurch der Schaft (2) und das Verankerungselement (4) axial zusammengehalten werden,
**dadurch gekennzeichnet, dass**
E) die axialen Arretiermittel (12) und die zusammenwirkenden Mittel (5;6) unabhängig voneinander wirken.

2. Knochenfixationsmittel (1) nach Anspruch 1, **dadurch gekennzeichnet, dass** der Schaft (2) und das Verankerungselement (4) axial voneinander lösbar sind.

3. Knochenfixationsmittel (1) nach Anspruch 1 oder 2, **dadurch gekennzeichnet, dass** die axialen Arretiermittel (12) zwischen dem Verankerungselement (4) und dem Schaft (2) elastisch ineinander einschnappbar ausgestaltet sind.

4. Knochenfixationsmittel (1) nach Anspruch 3, **dadurch gekennzeichnet, dass** die axialen Arretiermittel (12) radial elastische Zungen (13) mit Erhebungen (14) umfassen und dass die Erhebungen (14) in eine komplementäre, zur Längsachse (3) konzentrisch kreisförmige Nute (15) einrastbar sind.

5. Knochenfixationsmittel (1) nach Anspruch 4, **dadurch gekennzeichnet, dass** die Zungen (13) am Verankerungselement (4) angeordnet sind, und dass die Nute (15) am Schaft (2) angeordnet ist.

6. Knochenfixationsmittel (1) nach Anspruch 5, **dadurch gekennzeichnet, dass** die Erhebungen (14) konvex ausgebildet sind.

7. Knochenfixationsmittel (1) nach Anspruch 5 oder 6, **dadurch gekennzeichnet, dass** die Nuten (15) einen V-förmigen Querschnitt aufweisen.

8. Knochenfixationsmittel (1) nach Anspruch 1 oder 2, **dadurch gekennzeichnet, dass** die axialen Arretiermittel (12) mindestens einen quer zur Längsachse (3) fest angeordneten Arretierstift (21) umfassen, dessen Spitze in einen zur Längsachse (3) konzentrisch kreisförmigen Einstich (22) eingreifen.

9. Knochenfixationsmittel (1) nach Anspruch 1, **dadurch gekennzeichnet, dass** die axialen Arretiermittel (12) einen Sprengring (44) umfassen, welcher in einer zur Längsachse (3) konzentrischen ersten Ringnute (50) am Schaft (2) und in einer ebenfalls zur Längsachse (3) konzentrischen zweiten Ringnute (60) am Verankerungselement (4) eingespannt ist.

10. Knochenfixationsmittel (1) nach einem der Ansprüche 1 bis 9, **dadurch gekennzeichnet, dass** die zusammenwirkenden Mittel (5;6) einen Reibschluss zwischen Schaft (2) und Verankerungselement (4) ermöglichen.

11. Knochenfixationsmittel (1) nach Anspruch 10, **dadurch gekennzeichnet, dass** die Mittel (5;6) eine zur Längsachse (3) koaxiale, an der Verbindungsstelle von Schaft (2) und Verankerungselement (4) angeordnete Innenkonussegmente (19;20) und ein darin axial lösbar verkeilbares Konuselement (16) umfasst.

12. Knochentixationsmittel (1) nach Anspruch 11, **dadurch gekennzeichnet, dass** ein erstes Konussegment (19) im Schaft (2) und dazu korrespondierend, ein zweites Konussegment (20) im Verankerungselement (4) vorgesehen sind.

13. Knochenfixationsmittel (1) nach einem der Ansprüche 1 bis 10, **dadurch gekennzeichnet, dass** die zusammenwirkenden Mittel (5;6) am Verankerungselement (4) angeordnete, radial elastische Zungen (13) umfassen, welche mittels einer konischen Klemmschraube (73) gegen die Wand der Zentralbohrung (18) im Schaft (2) pressbar sind.

14. Knochenfixationsmittel (1) nach Anspruch 13, **dadurch gekennzeichnet, dass** die radial elastischen Zungen (13) Erhebungen (14) umfassen und dass die Erhebungen (14) in eine komplementäre, zur Längsachse (3) konzentrisch kreisförmige Nute (15) einrastbar sind.

15. Knochenfixationsmittel (1) nach einem der Ansprüche 1 bis 9, **dadurch gekennzeichnet, dass** die Mittel (5;6) einen rotativen Formschluss zwischen Schaft (2) und Verankerungselement (4) ermöglichen.

16. Knochenfixationsmittel (1) nach Anspruch 15, **dadurch gekennzeichnet, dass** der rotative Formschluss durch ineinander in Eingriff bringbare erste und zweite Verzahnungen (23;24) ausgebildet sind.

17. Knochenfixationsmittel (1) nach Anspruch 16, **dadurch gekennzeichnet, dass**
a) das Verankerungselement (4) eine an seinem festen, gegen den Schaft (2) gerichteten Ende (10) angeordnete, erste Verzahnung (23) umfasst; und
b) am Schaft (2) ein rotativ arretiertes, axial verschiebbares Fixationselement (56) mit einer zweiten, mit der ersten Verzahnung (23) in Eingriff bringbaren Verzahnung (24) angeordnet ist.

18. Knochenfixationsmittel (1) nach Anspruch 16, **dadurch gekennzeichnet, dass**
c) das Verankerungselement (4) ein rotativ arretiertes, axial verschiebbares Fixationselement (56) mit einer ersten Verzahnung (23) umfasst; und
d) am Schaft (2) eine zweite, mit der ersten Verzahnung (23) in Eingriff bringbare Verzahnung (24) angeordnet ist.

19. Knochenfixationsmittel (1) nach Anspruch 17 oder 18, **dadurch gekennzeichnet, dass** Fixationselement (56) mittels einer koaxial angeordneten Schraube (29) axial verschiebbar ist.

20. Knochenfixationsmittel (1) nach einem der Ansprüche 1 bis 19, **dadurch gekennzeichnet, dass** das Verankerungselement (4) eine Schraube mit einer Gewindesteigung G ist.

21. Knochenfixationsmittel (1) nach Anspruch 20, **dadurch gekennzeichnet, dass** die Gewindesteigung G grösser als 50 mm, vorzugsweise grösser als 80 mm ist.

22. Knochenfixationsmittel (1) nach einem der Ansprüche 1 bis 21, **dadurch gekennzeichnet, dass** der Schaft (2) einen unrunden Querschnitt aufweist.

23. Fixationsvorrichtung für die Osteosynthese mit einem Knochenfixationsmittel (1) nach einem der Ansprüche 1 - 22 **dadurch gekennzeichnet, dass** sie eine an einem Femur (48) befestigbare Knochenplatte (45) mit einer winklig anschliessenden Hülse (49) umfasst, wobei der Schaft (2) des Knochenfixationsmittel (1) in der Hülse (49) aufnehmbar ist.

24. Fixationsvorrichtung nach Anspruch 23, **dadurch gekennzeichnet, dass** die Hülse (49) innen einen unrunden Querschnitt aufweist und der Schaft (2) einen dazu korrespondierenden unrunden Querschnitt aufweist.

## Claims

1. Bone fixation means (1) comprising
A) a longitudinal shaft (2) with the longitudinal axis (3);
B) an anchoring element (4) with the identical longitudinal axis (3), which can be fastened axially to the shaft (2) and fixed in a bone; and
C) interacting means (5; 6) are provided at the shaft (2) and at the anchoring element (4) which alternatively permit or block rotation of the anchoring element (4) about the longitudinal axis (3) relative to the shaft (2); wherein
D) axial locking-in-position means (12) are disposed at the shaft (2) and at the anchoring element (4), by means of which the shaft (2) and the anchoring element (4) are held together axially,
**characterized in that**
E) the actions of the axial locking-in-position means (12) and the interacting means (5; 6) are independent of one another.

2. The bone fixation means (1) of claim 1, **characterized in that** the shaft (2) and the anchoring element (4) are detachable from one another axially.

3. The bone fixation means (1) of claim 1 or 2, **characterized in that** the axial locking-in-position means (12) between the anchoring element (4) and the shaft (2) are configured so that they can be snapped elastically into one another.

4. The bone fixation means (1) of claim 3, **characterized in that** the axial locking-in-position means (12) comprise radially elastic blades (13) with elevations (14) and that the elevations (14) can be snapped into a complementary, circular groove (15), which is concentric with the longitudinal axis (3).

5. The bone fixation means (1) of claim 4, **characterized in that** the blades (13) are disposed at the anchoring element (4) and that the groove (15) is disposed at the shaft (2).

6. The bone fixation means (1) of claim 5, **characterized in that** the elevations (14) are constructed convex.

7. The bone fixation means (1) of claims 5 or 6, **characterized in that** the grooves (15) have a V-shaped cross section.

8. The bone fixation means (1) of claim 1 or 2, **characterized in that** the axial locking-in-position means (12) comprise at least one locking-in-position pin (21), which is fixed transversely to the longitudinal axis (3) and the tip of which engages a circular groove (22), which is concentric with the longitudinal axis (3).

9. The bone fixation means (1) of claim 1, **characterized in that** the axial locking-in-position means (12) comprise a retaining ring (44), which is clamped in a first annular groove (50) at the shaft (2), which is concentric with the longitudinal axis (3), and in a second annular groove (60) at the anchoring element (4), which is also concentric with the longitudinal axis (3).

10. The bone fixation means (1) of one of the claims 1 to 9, **characterized in that** the interacting means (5; 6) allow a frictional connection between the shaft (2) and the anchoring element (4).

11. The bone fixation means (1) of claim 10, **characterized in that** the means (5; 6) comprises an inner conical segment (19; 20), which is disposed coaxially with the longitudinal axis (3) at the place connecting the shaft (2) and the anchoring element (4), and a conical element (16), which can be wedged axially detachably therein.

12. The bone fixation means (1) of claim 11, **characterized in that** a first conical segment (19) is provided in the shaft (2) and, corresponding thereto, a second conical segment (20) is provided in the anchoring element (4).

13. The bone fixation means (1) of one of the claims 1 to 10, **characterized in that** the interacting means (5; 6) comprise radially elastic blades (13), which are disposed at the anchoring element (4) and can be pressed by means of a conical locking screw (73) against the wall of the central borehole (18) in the shaft (2).

14. The bone fixation means (1) of claim 13, **characterized in that** the radially elastic blades (13) comprise elevations (14) and that the elevations (14) can be snapped into a complementary circular groove (15), which is concentric with the longitudinal axis (3).

15. The bone fixation means (1) of one of the claims 1 to 9, **characterized in that** the means (5; 6) allow a rotationally positive connection between the shaft (2) and the anchoring element (4).

16. The bone fixation means (1) of claim 15, **characterized in that** the rotational positive connection is formed by a first and a second toothing (23; 24), which can be brought into engagement with one another.

17. The bone fixation means (1) of claim 16, **characterized in**
a) **that** the anchoring element (4) comprises a first toothing (23), which is disposed at its fixed end (10), which is directed at against the shaft (2), and
b) **that** a rotationally locked-in-position, axially displaceable fixation element (56) with a second toothing (24), which can be brought into engagement with the first toothing (23), is disposed at the shaft (2).

18. The bone fixation means (1) of claim 16, **characterized in**
c) that the anchoring element (4) comprises a rotationally locked-in-position, axially displaceable fixation element (56) with a first toothing (23) and
d) that a second toothing (24), which can be brought into engagement with the first toothing (23), is disposed at the shaft (2).

19. The bone fixation means (1) of claims 17 or 18, **characterized in that** the fixation element (56) can be shifted axially by means of a screw (29), which is disposed coaxially.

20. The bone fixation means (1) of one of the claims 1 to 19, **characterized in that** the anchoring element (4) is a screw with a thread pitch of G.

21. The bone fixation means (1) of claim 20, **characterized in that** the thread pitch G is larger than 50 mm and preferably larger than 80 mm.

22. The bone fixation means (1) of one of the claims 1 to 21, **characterized in that** the shaft (2) has a noncircular cross section.

23. Fixation device for the osteosynthesis with a bone fixation means (1) of one of the claims 1 to 22, **characterized in that** it comprises a bone plate (45), which can be fastened to a femur (48) and has an angularly adjoining sleeve (49), wherein the shaft (2) of the bone fixation means (1) is receivable in the sleeve (49).

24. The fixation device of claim 23, **characterized in that** the sleeve (49) has an inner noncircular cross-section and that the shaft (2) has a noncircular cross section corresponding thereto.

## Revendications

1. Moyen de fixation d'os (1) comportant
A) une tige longitudinale (2) d'axe longitudinal (3) ;
B) un élément d'ancrage (4), de même axe longitudinal (3), qui peut être immobilisé axialement sur la tige (2) et qui peut être fixé dans un os ; et
C) des moyens coopérants (5 ; 6) étant prévus sur la tige (2) et l'élément d'ancrage (4), lesquels moyens autorisent ou bloquent au choix une rotation de l'élément d'ancrage (4) par rapport à la tige (2) autour de l'axe longitudinal (3),
D) des moyens d'arrêt axial (12) étant disposés au niveau de la tige (2) et au niveau de l'élément d'ancrage (4) de sorte que la tige (2) et l'élément d'ancrage (4) sont maintenus axialement ensemble,
**caractérisé en ce que**
E) les éléments d'arrêt axial (12) et les moyens coopérants (5 ; 6) agissent indépendamment l'un de l'autre.

2. Moyen de fixation d'os (1) selon la revendication 1, **caractérisé en ce que** la tige (2) et l'élément d'ancrage (4) peuvent être détachés axialement l'un de l'autre.

3. Moyen de fixation d'os (1) selon la revendication 1 ou 2, **caractérisé en ce que** les moyens d'arrêt axial (12) sont conformés de façon à pouvoir être encliquetés élastiquement l'un dans l'autre entre l'élément d'ancrage (4) et la tige (2).

4. Moyen de fixation d'os (1) selon la revendication 3, **caractérisé en ce que** les moyens d'arrêt axial (12) comportent des languettes (3) radialement élastiques et dotées de saillies (14) et **en ce que** les saillies (14) peuvent être encliquetées dans une gorge circulaire complémentaire (15) disposée concentriquement à l'axe longitudinal (3).

5. Moyen de fixation d'os (1) selon la revendication 4, **caractérisé en ce que** les languettes (13) sont disposées au niveau de l'élément d'ancrage (4), et **en ce que** la gorge (15) est ménagée au niveau de la tige (2).

6. Moyen de fixation d'os (1) selon la revendication 5, **caractérisé en ce que** les saillies (14) présentent une conformation convexe.

7. Moyen de fixation d'os (1) selon la revendication 5 ou 6, **caractérisé en ce que** les gorges (15) présentent une section en forme de V.

8. Moyen de fixation d'os (1) selon la revendication 1 ou 2, **caractérisé en ce que** les moyens d'arrêt axial (12) comportent au moins une tige d'arrêt (21) qui est disposée de façon fixe transversalement à l'axe longitudinal (3) et dont la pointe s'engage dans une encoche circulaire (22) ménagée concentriquement à l'axe longitudinal (3).

9. Moyen de fixation d'os (1) selon la revendication 1, **caractérisé en ce que** les moyens d'arrêt axial (12) comportent un circlips (44) qui est enserré au niveau de la tige (2) dans une première gorge annulaire (50) concentrique à l'axe longitudinal (3) et au niveau de l'élément d'ancrage (4) dans une seconde gorge annulaire (60) également concentrique à l'axe longitudinal (3).

10. Moyen de fixation d'os (1) selon l'une des revendications 1 à 9, **caractérisé en ce que** les moyens coopérants (5 ; 6) permettent une liaison par friction entre la tige (2) et l'élément d'ancrage (4).

11. Moyen de fixation d'os (1) selon la revendication 10, **caractérisé en ce que** les moyens (5 ; 6) comportent des segments coniques intérieurs (19 ; 20) coaxiaux à l'axe longitudinal (3) et disposés au niveau de la liaison de la tige (2) et de l'élément d'ancrage (4) et un élément conique (16) qui peut être calé de façon amovible axialement à l'intérieur.

12. Moyen de fixation d'os (1) selon la revendication 11, **caractérisé en ce qu'**un premier segment conique (19) est prévu dans la tige (2) et, en correspondance de celui-ci, un second segment conique (20) est prévu dans l'élément d'ancrage (4).

13. Moyen de fixation d'os (1) selon l'une des revendications 1 à 10, **caractérisé en ce que** les moyens coopérants (5 ; 6) comportent des languettes radialement élastiques (13) disposées au niveau de l'élément d'ancrage (4), lesquelles languettes peuvent être pressées au moyen d'une vis de serrage conique (73) contre la paroi de l'alésage central (18) ménagé dans la tige (2).

14. Moyen de fixation d'os (1) selon la revendication 13, **caractérisé en ce que** les languettes radialement élastiques (13) comportent des saillies (14) et **en ce que** les saillies (14) peuvent être encliquetées dans une gorge circulaire complémentaire (15) ménagée concentriquement à l'axe longitudinal (3).

15. Moyen de fixation d'os (1) selon l'une des revendications 1 à 9, **caractérisé en ce que** les moyens (5 ; 6) permettent une liaison en rotation par complémentarité de formes entre la tige (2) et l'élément d'ancrage (4).

16. Moyen de fixation d'os (1) selon la revendication 15, **caractérisé en ce que** la liaison en rotation par complémentarité de formes est configurée en première et seconde dentures (23 ; 24) pouvant être amenées en prise l'une dans l'autre.

17. Moyen de fixation d'os (1) selon la revendication 16, **caractérisé en ce que**
a) l'élément d'ancrage (4) comporte à son extrémité fixe (10) dirigée vers la tige (2) une première denture (23); et
b) au niveau de la tige (2) est disposé un élément de fixation (56), bloqué en rotation et apte à coulisser axialement, qui est doté d'une seconde denture (24) pouvant être amenée en prise avec la première denture (23).

18. Moyen de fixation d'os (1) selon la revendication 16, **caractérisé en ce que**
c) l'élément d'ancrage (4) comporte un élément de fixation (56) bloqué en rotation, apte à coulisser axialement et doté d'une première denture (23) ; et
d) au niveau de la tige (2) est disposée une seconde denture (24) pouvant être amenée en prise avec la première denture (23).

19. Moyen de fixation d'os (1) selon la revendication 17 ou 18, **caractérisé en ce que** l'élément de fixation (56) est apte à coulisser axialement au moyen d'une vis (29) disposée coaxialement.

20. Moyen de fixation d'os (1) selon l'une des revendications 1 à 19, **caractérisé en ce que** l'élément d'ancrage (4) est une vis de pas G.

21. Moyen de fixation d'os (1) selon la revendication 20, **caractérisé en ce que** le pas G est supérieur à 50 mm, de préférence supérieur à 80 mm.

22. Moyen de fixation d'os (1) selon l'une des revendications 1 à 21, **caractérisé en ce que** la tige (2) présente une section non circulaire.

23. Dispositif de fixation pour ostéosynthèse comportant un moyen de fixation d'os (1) selon l'une des revendications 1 à 22, **caractérisé en ce qu'**il comporte une plaque pour os (45) qui peut être fixée à un fémur (48) et à laquelle un manchon (49) est raccordé angulairement, la tige (2) du moyen de fixation d'os (1) pouvant être reçue dans le manchon (49).

24. Dispositif de fixation selon la revendication 23, **caractérisé en ce que** le manchon (49) présente à l'intérieur une section non circulaire et la tige (2) présente une section non circulaire correspondante.
